# EUROPÄISCHE PATENTSCHRIFT

(11) **EP 0 645 372 B1**
(45) Veröffentlichungstag und Bekanntmachung des Hinweises auf die Patenterteilung: **04.12.1996**
(21) Anmeldenummer: 94113853.9
(22) Anmeldetag: 03.09.1994
(51) Int. Cl.: C07C 263/18, C07C 265/14

(54) **Stabile nach phosgenfreien Verfahren erhältliche Polyisocyanatzusammensetzungen und ein Verfahren zu ihrer Herstellung**
Stable polyisocyanate mixtures prepared by phosgen-free processes and a process for their preparation
Mélanges de polyisocyanates stables préparés par un procédé sans phosgène et un procédé pour leur préparation

(30) Priorität: 13.09.1993 DE 4331085
(43) Veröffentlichungstag der Anmeldung: 29.03.1995
(73) Patentinhaber: BASF Aktiengesellschaft, 67063 Ludwigshafen (DE)
(72) Erfinder: Laqua, Gerhard, Dr., D-68167 Mannheim (DE); Bruchmann, Bernd, Dr., D-67069 Ludwigshafen (DE); Wolff, Stefan, Dr., D-67117 Limburgerhof (DE)

(56) Entgegenhaltungen:
- EP-A- 0 203 874
- EP-A- 0 355 443
- EP-A- 0 445 608
- EP-A- 0 531 803
- US-A- 3 247 236

## Beschreibung

Nach phosgenfreien Verfahren, vorzugsweise durch thermische Spaltung von (cyclo)aliphatischen Polycarbaminsäureestern erhältliche (cyclo)aliphatische Polyisocyanatzusammensetzungen werden stabilisiert durch den Zusatz von Kohlendioxid.

Organische Polyisocyanate, wie z.B. aromatische, cycloaliphatische und aliphatische di- und höherwertige Polyisocyanate können nach verschiedenartigen Verfahren hergestellt werden (Annalen der Chemie 562 (1949), Seiten 75ff). Technisch insbesondere bewährt hat sich die Herstellung von organischen Polyisocyanaten durch Phosgenierung von organischen Polyaminen zu den entsprechenden Polycarbaminsäurechloriden und deren thermische Spaltung in organische Polyisocyanate und Chlorwasserstoff, so daß zur Zeit ausschließlich dieses Herstellungsverfahren industriell genutzt wird.

Problematisch bei dieser Verfahrensweise sind der hohe Umsatz von Chlor über Phosgen und Carbaminsäurechlorid in Chlorwasserstoff, die Toxizität des Phosgens sowie die damit verbundenen kostspieligen Sicherheitsvorkehrungen, die Korrosivität der Reaktionsmischung, die Labilität der üblicherweise eingesetzten Lösungsmittel und die Bildung von chlorhaltigen und chlorfreien Nebenprodukten, die die physikalischen Eigenschaften, wie z.B. die Farbe, die Viskosität und den Dampfdruck, und chemischen Eigenschaften, wie z.B. Reaktivität, Lagerbeständigkeit u.a. der Polyisocyanate sowie die mechanischen Eigenschaften der aus solchen Polyisocyanaten hergestellten Polyisocyanat-Polyadditionsprodukten entscheidend mitbestimmen.

Es hat daher nicht an Versuchen gefehlt, organische, vorzugsweise aromatische Polyisocyanate ohne die Verwendung von Phosgen, d.h. nach phosgenfreien Verfahren, herzustellen.

Nach Angaben der EP-B-0 126 299 (US-A-4 596 678), EP-B-0 126 300 (US-A-4 596 679) und EP-A-0 355 443 (US-A-5 087 739) können (cyclo)aliphatische Diisocyanate, wie 1,6-Hexamethylen-diisocyanat (HDI) und/oder isomere aliphatische Diisocyanate mit 6 Kohlenstoffatomen im Alkylenrest und 1-Isocyanato-3-isocyanato-methyl-3,5,5-trimethyl-cyclohexan (Isophoron-diisocyanat bzw. IPDI) in Kreislaufverfahren hergestellt werden durch Umsetzung der (cyclo)aliphatischen Diamine mit Harnstoff und Alkoholen und gegebenenfalls N-unsubstituierten Carbaminsäureestern, Dialkylcarbonaten und anderen aus dem Reaktionsprozeß zurückgeführten Nebenprodukten zu (cyclo)aliphatischen Bis-carbaminsäureestern und deren thermische Spaltung in die entsprechenden Diisocyanate und Alkohole.

Je nach Art des Herstellungsverfahrens enthalten die organischen Polyisocyanate unterschiedliche Nebenprodukte mit vielfach unbekannter Struktur, wobei insbesondere die chlorhaltigen Nebenprodukte die Lagerbeständigkeit, Reaktivität und Farbe der Zusammensetzung beeinflussen.

Nach Angaben der US-A-3 330 849 können z.B. organische Polyisocyanate durch den Zusatz von Sulfonylisocyanaten gegen eine Verfärbung und Niederschlagsbildung stabilisiert werden. Durch den Zusatz von Metallnaphthenaten, wie z.B. Cadmium-, Kobalt-, Kupfer-, Blei-, Mangan- oder Zinknaphthenat, kann der hydrolysierbare Chlorgehalt von Isocyanaten gemäß US-A-3 373 182 reduziert werden. Die US-A-3 384 653 und US-A-3 449 256 beschreiben die Verbesserung der Lagerbeständigkeit von 4,4'-Di-phenylmethan-diisocyanat durch eine Behandlung bei 160° bis 250°C mit Trialkylphosphaten. Der Gehalt an hydrolysierbaren Chlorverbindungen kann gemäß US-A-3 458 558 bei organischen Isocyanaten auch mit Kupfer, Silber, Nickel, Eisen und Zink bei Temperaturen über 100°C erniedrigt werden. Nach Angaben der US-A-3 479 393 stabilisieren Trialkylaminborane Isocyanate gegen eine Verfärbung. Gemäß US-A-3 535 359 eignen sich Ortho-carbonsäureester zur Stabilisierung von organischen Isocyanaten gegen eine Viskositätserhöhung. Diphenylmethan-diisocyanat enthaltende Polyisocyanatmischungen können nach Angaben der US-A-3 585 229 durch Zusatz von Diphenyl-decylphosphit entfärbt werden. Gegen eine Zersetzung können organische Polyisocyanate gemäß US-A-3 692 813 mit Hilfe von Oxycarbonylisocyanaten mit mindestens einer Gruppe der Formel -O-CO-NCO stabilisiert werden. Zur Stabilisierung von organischen Polyisocyanaten gegen eine Verfärbung kann nach Angaben der US-A-3 715 381 2,6-Di-tert.-butyl-p-kresol eingesetzt werden. Di-phenylmethan-diisocyanate können gemäß US-A-3 970 680 auch durch Zugabe von tertiären Aminen stabilisiert werden. Zur Reinigung von organischen Isocyanaten können diese gemäß US-A-4 065 362 bei Temperaturen über 100°C mit einem Metallsalz des Mercaptobenzthiazol, einem Metallsalz von alkylsubstituierten Dithiocarbaminsäuren, einem alkylsubstituierten Phenol, einem Thio-bisphenol oder einem Triarylphosphit behandelt werden. Nach Angaben der US-A-3 247 236 können durch Umsetzung von Diaminen mit Phosgen hergestellte und durch Destillation gereinigte Diisocyanate durch Zusatz von Kohlendioxid oder Schwefeldioxid stabilisiert werden. Nachteilig an diesem Verfahren ist die gute Löslichkeit des Schwefeldioxids im Polyisocyanat und die Ausbildung von Verfärbungen während der Lagerung. Nachteilig ist ferner, daß die mit Kohlendioxid stabilisierte Polyisocyanate nur unter Überwindung von beträchtlichen Schwierigkeiten Trimerisierungsreaktionen zugänglich sind. Zur Trimerisierung von Hexamethylen-diisocyanat muß z.B. gemäß DE-A-3 806 276 dieses vom Kohlendioxid bis auf einen Restgehalt von kleiner als 20 ppm befreit werden. Die US-A-3 247 236 und die vorgenannten anderen Patentpublikation vermitteln keinerlei Lehre hinsichtlich einer Stabilisierung von nach phosgenfreien Verfahren hergestellten organischen Polyisocyanaten.

Nach phosgenfreien Verfahren, insbesondere durch thermische Spaltung von (cyclo)aliphatischen Polycarbaminsaureestern erhältliche (cyclo)aliphatische Polyisocyanate sind nicht lagerbeständig. Ihre Instabilität ist auf das Fehlen von hydrolysierbaren Chlorverbindungen und auf das Vorliegen von katalytisch wirkenden Verunreinigungen unbekannter Struktur zurückzuführen, die z.B. die Bildung von Oligomeren begünstigen. Bei tiefen Temperaturen, z.B. bei +5°C und darunter, neigt z.B. Hexamethylen-diisocyanat (HDI) zur Bildung linearer HDI-Oligomerer der Formel

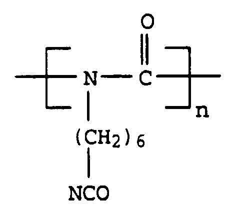

Die Molekulargewichtserhöhung, verbunden mit einer Viskositätszunahme, kann zum Gelieren des Polyisocyanats, z.B. HDI's führen. Derartige Produkte sind nur schwierig handhabbar, nicht mehr reproduzierbar zu Polyisocyanat-Polyadditionsprodukten umsetzbar und müssen daher verworfen werden. Bei höheren Lagertemperaturen nimmt z.B. die Reaktivität des nach phosgenfreien Verfahren hergestellten HDI, insbesondere bei der mit quaternären Ammoniumhydroxidverbindungen katalysierten Trimerisierungsreaktion, stark ab. Man erhält intensiv gefärbte, Isocyanuratgruppen aufweisende Polyisocyanate, die insbesondere zur Verwendung als Lackrohstoff nicht mehr verwertbar sind.

Die Aufgabe der vorliegenden Erfindung bestand darin, nach phosgenfreien Verfahren hergestellte (cyclo)aliphatische Polyisocyanate durch geeignete Maßnahmen zu stabilisieren, ohne die Reaktivität der Polyisocyanate nachteilig zu beeinflussen. Die Oligomerisierung der Polyisocyanate bzw. eine Viskositätserhöhung der Polyisocyanatzusammensetzung sollte ebenso verhindert werden, wie ihre Verfärbung während der Lagerung.

Diese Aufgabe konnte überraschenderweise mit Kohlendioxid als Stabilisator gelöst werden.

Gegenstand der Erfindung sind somit stabile (cyclo)aliphatische Polyisocyanatzusammensetzungen, die enthalten
i) ein nach einem phosgenfreien Verfahren hergestelltes, vorzugsweise durch thermische Spaltung von (cyclo)aliphatischen Polycarbaminsäureestern erhältliches (cyclo)aliphatisches Polyisocyanat und
ii) Kohlendioxid.

Durch den Zusatz von Kohlendioxid konnte überraschenderweise die Reaktivität der (cyclo)aliphatischen Polyisocyanate und ihre Farbzahl über eine Lagerzeit von mindestens 12 Wochen bei Raumtemperatur stabilisiert werden. Überraschend und nicht vorhersehbar war ferner die problemlose Trimerisierung der (cyclo)aliphatischen Polyisocyanate mit quaternären Ammoniumverbindungen, wie z.B. N,N,N-Trimethyl-N-(2-hydroxypropyl)ammonium-2-ethylhexanoat als Trimerisierungskatalysator. Im Gegensatz zu der Lehre der DE-A-38 06 276, nach der in Gegenwart von hohen Kohlendioxidgehalten aus monomeren, durch Phosgenierung hergestellten Polyisocyanate isocyanuratgruppenhaltige Polyisocyanate mit hohen Farbzahlen erhalten werden, wird bei der Trimerisierung der erfindungsgemäßen (cyclo)aliphatischen Polyisocyanatzusammensetzungen die Farbzahl weiter reduziert und dadurch zusätzlich verbessert.

Die erfindungsgemäßen Polyisocyanatzusammensetzungen können beliebige (cyclo)aliphatische Polyisocyanate enthalten mit der Maßgabe, daß diese nach geeigneten Verfahren in Abwesenheit von Phosgen hergestellt wurden. Vorzüglich bewährt haben sich und daher vorzugsweise Verwendung finden (cyclo)aliphatische Polyisocyanate, die durch thermische Spaltung von (cyclo)aliphatischen Polycarbaminsaureestern erhältlich sind. Geeignete aliphatische Polyisocyanate besitzen vorteilhafterweise 3 bis 16 C-Atome, vorzugsweise 4 bis 12 C-Atome im linearen oder verzweigtkettigen Alkylenrest und geeignete cycloaliphatische Polyisocyanate vorteilhafterweise 4 bis 18 C-Atome, vorzugsweise 6 bis 15 C-Atome im Cycloalkylenrest. Beispielhaft genannt seien: 1,4-Diisocyanato-butan, 2-Ethyl-1,4-diisocyanato-butan, 1,5-Diisocyanatopentan, 2-Methyl-1,5-diisocyanato-pentan, 2,2-Dimethyl-1,5-diisocyanato-pentan, 2-Ethyl-2-propyl-1,5-diisocyanato-pentan, 2-Ethyl-2-butyl-1,5-diisocyanato-pentan, 2-Alkoxymethylen-1,5-diisocyanato-pentan, 1,6-Hexamethylen-diisocyanat, 2,4,4- bzw. 2,2,4-Trimethyl-1,6-hexamethylen-diisocyanat, 1,7-Diisocyanato-heptan, 1,8-Diisocyanato-octan, 1,10-Diisocyanato-decan, 1,12-Diisocyanato-dodecan, 4,4'-Diisocyanato-dicyclohexylmethan, 2,4'-Diisocyanato-dicyclohexylmethan sowie Gemische der Diisocyanato-dicyclohexylmethan-Isomeren, 1,3-Diisocyanato-cyclohexan sowie Isomerengemische von Diisocyanato-cyclohexanen und l-Isocyanato-3-isocyanatomethyl-3,5,5-trimethylcyclohexan. Als (cyclo)aliphatische Polyisocyanate vorzugsweise verwendet werden l,6-Hexamethylen-diisocyanat, isomere aliphatische Diisocyanate mit 6 Kohlenstoffatomen im Alkylenrest sowie Mischungen davon und l-Isocyanato-3-isocyanatomethyl-3,5,5-trimethylcyclohexan.

Wie bereits dargelegt wurde, werden die (cyclo)aliphatischen Diisocyanate vorzugsweise durch thermische Spaltung der entsprechenden Dicarbaminsaureester hergestellt. Diese Spaltung kann beispielsweise bei Temperaturen von 150 bis 300°C, vorzugsweise von 180 bis 250°C und Drücken von 0,001 bis 2 bar, vorzugsweise von 1 bis 200 mbar in Abwesenheit oder vorzugsweise Gegenwart von Katalysatoren in geeigneten Spaltreaktoren, wie z.B. Dünnschicht- oder vorzugsweise Heizkerzenverdampfern nach der EP-A-0 524 554 durchgeführt werden. Die bei der Spaltung entstehenden Diisocyanate und Alkohole können beispielsweise durch fraktionierte Kondensation oder vorzugsweise durch Rektifikation getrennt und die Diisocyanate z.B. durch Destillation zusätzlich gereinigt werden.

Zur Stabilisierung der nach phosgenfreien Verfahren, vorzugsweise durch thermische Spaltung von (cyclo)aliphatischen Polycarbaminsäureestern, erhältlichen (cyclo)aliphatischen Polyisocyanate kann das Kohlendioxid in Mengen bis zum Sättigungspunkt des Kohlendioxids in dem speziellen Polyisocyanat verwendet werden, ohne daß dadurch seine physikalischen oder chemischen Eigenschaften, insbesondere seine Fähigkeit zur partiellen oder vollständigen Trimerisation, nachteilig beeinflußt werden. Aus wirtschaftlichen Gründen wird das Kohlendioxid zweckmäßigerweise in einer Menge bis 0,3 Gew.-% vorzugsweise von 0,004 bis 0,3 Gew.-% und insbesondere von 0,05 bis 0,15 Gew.-%, bezogen auf das Gesamtgewicht der Polyisocyanatzusammensetzung verwendet.

Das Kohlendioxid kann z.B. pulverförmig in die nach phosgenfreien Verfahren hergestellten (cyclo)aliphatischen Polyisocyanate, zweckmäßigerweise unter Rühren, eingebracht werden. Nach einer anderen Verfahrensvariante kann das Kohlendioxid unter Druck und zweckmäßigerweise unter Rühren in das (cyclo)aliphatische Polyisocyanat in der erforderlichen Menge eingepreßt werden. Nach einer bevorzugten Ausführungsform läßt man das gasförmige Kohlendioxid in der erforderlichen Menge durch das (cyclo)aliphatische Polyisocyanat perlen, wobei im Rahmen der Lagerzeit zusätzlich Kohlendioxid eingebracht werden kann. Zweckmäßigerweise wird das Kohlendioxid über geeignete Düsen in die Lagerbehälter eingebracht.

Nach dem erfindungsgemäßen Verfahren stabilisierte (cyclo)aliphatische Polyisocyanat-Zusammensetzungen, vorzugsweise die 1,6-Hexamethylen-diisocyanat- und 1-Isocyanato-3-isocyanatomethyl-3,5,5-trimethyl-cyclohexan-Zusammensetzungen können auf diese Weise mehr als 12 Wochen gelagert werden.

Die nach phosgenfreien Verfahren, vorzugsweise durch thermische Spaltung von (cyclo)aliphatischen Polycarbaminsäureestern, erhältlichen (cyclo)aliphatischen Polyisocyanatzusammensetzungen können durch den Zusatz allein von Kohlendioxid stabilisiert werden. Es ist jedoch auch möglich, das Kohlendioxid in Verbindung mit anderen stabilisierend wirkenden Verbindungen zu verwenden.

Als zusätzliche Stabilisatoren eignen sich beispielsweise phenolische Antioxidantien, so daß diese vorzugsweise mitverwendet werden. Mitverwendet werden können z.B. auch phosphorhaltige Stabilisatoren.

Als phenolische Antioxidantien eignen sich beispielsweise Verbindungen, die mindestens eine sterische gehinderte phenolische Gruppierung besitzen. Als Beispiele für solche Antioxidantien seien genannt:
2,6-Di-t-butyl-4-methylphenol, 2,4,6-Tri-t-butylphenol, 2,2'-Methylen-bis-(4-methyl-6-t-butylphenol), 2,2'-Thio-bis-(4-methyl-6-t-butylphenol), 4,4'-Thio-bis-(3-methyl-6-t-butylphenol), 4,4'-Butyliden-bis-(6-t-butyl-3-methylphenol), 4,4'-Methyliden-bis-(2,6-di-t-butylphenol), 2,2'-Methylen-bis-[4-methyl-6-(1-methylcyclohexyl)phenol], Tetrakis[methylen-3-(3,5-di-t-butyl-4-hydroxyphenyl)-propionat]methan, 1,3,5-Trimethyl-2,4,6-tris(3,5-di-t-butyl-4-hydroxybenzyl)benzol, N,N'-Hexamethylen-bis-(3,5-di-t-butyl-4-hydroxy-hydrozimtsäureamid), octadecyl-3-(3,5-di-t-butyl-4-hydroxyphenyl)-propionat, 1,3,5-Tris-(3,5-di-t-butyl-4-hydroxybenzyl)-isocyanurat, 1,1,3-Tris-(5-t-butyl-4-hydroxy-2-methylphenyl)butan, 1,3,5-Tris-(3,5-di-t-butyl-4-hydroxybenzyl)mesitylen, Ethylenglykol-bis[3,3-bis-(3'-t-butyl-4'-hydroxyphenyl)butyrat], 2,2'-Thiodiethyl-bis-3-(3,5-di-t-butyl-4-hydroxyphenyl)propionat, Di-(3-t-butyl-4'-hydroxy-5-methylphenyl)dicyclopentadien, 2,2'-Methylen-bis-(4-methyl-6-cyclohexylphenol), 1,6-Hexandiolbis-(3,5-di-t-butyl-4-hydroxyphenyl)propionat, 2,4-Bis(n-octylthio)-6-(4-hydroxy-3,5-di-t-butylanilino)-1,3,5-triazin, 3,5-Di-t-butyl-4-hydroxybenzyl-phosphonsäure-diethylester und Triethylenglykol-bis-3-(t-butyl-4-hydroxy-5-methylphenyl)-propionat.

Vorzüglich bewährt haben sich und daher vorzugsweise verwendet werden z.B. Octadecyl-3-(3,5-di-t-butyl-4-hydroxyphenyl)propionat, 2,2'-Methylen-bis-(4-methyl-6-cyclohexylphenol), 2,2'-Methylen-bis-(4-methyl-6-t-butylphenol), Triethylen-glykol-bis-3-(t-butyl-4-hydroxy-5-methylphenyl)-propionat, Tetra-kis[methylen-3-(3,5-di-t-butyl-4-hydroxyphenyl)-propionat]methan und insbesondere 2,6-Di-t-butyl-4-methylphenol.

Als geeignete phosphorhaltige Stabilisatoren seien beispielhaft genannt: Distearylpentaerythritdiphosphit, Tris-(nonylphenyl)phosphit, Tetrakis-(2,4-di-t-butylphenyl-4,4'-biphenylen-diphosphonit, Tris-(2,4-di-t-butylphenyl)phosphit, Neopentylglykoltriethylenglykoldiphosphit, Diisodecylpentaerythritdiphosphit, Tristearylphosphit, Trilaurylphosphit und Triphenylphosphit.

Die zusätzlichen Stabilisatoren, vorzugsweise die beispielhaft genannten phenolischen Antioxidantien und/oder phosphorhaltigen Verbindungen können z.B. in Mengen von 0,001 bis 0,1 Gew.-%, vorzugsweise von 0,005 bis 0,05 Gew.-%, bezogen auf das Gewicht der Polyisocyanatzusammensetzung verwendet werden.

Die phosgenfrei hergestellten, vorzugsweise durch thermische Spaltung von Polycarbaminsaureestern erhältlichen (cyclo)aliphatischen Polyisocyanate werden somit bevorzugt stabilisiert durch eine Kombination aus Kohlendioxid, phenolischen Antioxidantien und/oder phosphorhaltigen Verbindungen und enthalten, bezogen auf das Gesamtgewicht, zweckmäßigerweise bis 0,3 Gew.-%, vorzugsweise 0,004 bis 0,3 Gew.-% und insbesondere 0,05 bis 0,15 Gew.-% Kohlendioxid, 0 bis 0,1 Gew.-%, vorzugsweise 0,001 bis 0,05 Gew.-% und insbesondere 0,01 bis 0,03 Gew.-% mindestens eines phenolischen Antioxidants und 0 bis 0,1 Gew.-%, vorzugsweise 0,001 bis 0,05 Gew.-% und insbesondere 0,01 bis 0,03 Gew.-% mindestens einer phosphorhaltigen Verbindung.

### Beispiele

### Beispiel 1

Eine durch thermische Zersetzung von Hexamethylen-dibutylurethan-1,6 hergestellte Hexamethylen-diisocyanat-1,6 (HDI)-Zusammensetzung wurde bei 23°C 5 Minuten mit trockenem Kohlendioxid begast. Die HDI-Probe, die einen Kohlendioxidgehalt von ungefähr 1000 ppm aufwies, wurde in einem Behälter aus V2A-Stahl bei 40°C gelagert.

### Vergleichsbeispiel I

Ein Teil der obengenannten HDI-Zusammensetzung wurde unter denselben Bedingungen, jedoch in Abwesenheit von Kohlendioxid, gelagert.

### Herstellung von Isocyanuratgruppen enthaltenden Polyisocyanatmischungen:

Nach einer Lagerzeit von 30 Tagen wurden die vorgenannten HDI-Zusammensetzungen in Gegenwart von 500 ppm N,N,N-Trimethyl-N-(2-hydroxypropyl)ammonium-2-ethylhexanoat bei 80°C unter Rühren trimerisiert. Nach einer Stunde wurde die Reaktion durch Zugabe von Dibutylphosphat gestoppt.

An den Reaktionsmischungen wurden die folgenden Eigenschaften gemessen:

| | Beispiel 1 | Vergleichsbeispiel I |
|---|---|---|
| HDI-Gehalt nach der Lagerung. [Gew.-%] Restliche Bestandteile zu 100 Gew.-%: | > 99,9 Oligomere | 99,62 Oligomere |
| NCO-Gehalt der Reaktionsmischung nach der Trimerisierung [Gew.-%] | 39,3 | 47,8 |
| Farbzahl der Reaktionsmischung nach der Trimerisierung [Hazen] | 169 | 412 |

Die Ergebnisse zeigen, daß die erfindungsgemäße HDI-Zusammensetzung nach Beispiel 1 nach der Lagerung unter Kohlendioxid einen geringeren Oligomerengehalt besaß, d.h. stabiler war, und die Reaktionsmischung nach der Trimerisierung eine niedrigere Farbzahl, d.h. eine hellere Farbe, aufwies als die HDI-Zusammensetzung nach dem Vergleichsbeispiel I.

### Beispiel 2

Eine durch thermische Zersetzung von Hexamethylen-dibutylurethan-1,6 hergestellte HDI-Zusammensetzung wurde bei 23°C 5 Minuten mit trockenem Kohlendioxid begast und danach mit 100 mol-ppm 2,6-Di-tert.-butyl-4-methylphenol versetzt. Die HDI-Zusammensetzung, die einen Kohlendioxidgehalt von ungefähr 1000 ppm besaß, wurde in einem Behälter aus V2A-Stahl bei 40°C gelagert.

### Vergleichsbeispiel II

Ein Teil der vorgenannten HDI-Zusammensetzung wurde lediglich mit 100 mol-ppm 2,6-Di-tert.-butyl-4-methylphenol stabilisiert und analog Beispiel 2 gelagert.

### Herstellung von Isocyanuratgruppen enthaltenden Polyisocyanatmischungen

Nach einer Lagerzeit von 30 Tagen wurden die HDI-Zusammensetzungen nach Beispiel 2 und Vergleichsbeispiel II in Gegenwart von 500 ppm N,N,N-Trimethyl-N-(2-hydroxypropyl)ammonium-2-ethylhexanoat bei 80°C unter Rühren trimerisiert. Nach einer Stunde wurde die Reaktion durch Zugabe von Dibutylphosphat gestoppt.

An den Reaktionsmischungen wurden die folgenden Meßergebnisse ermittelt:

| | Beispiel 2 | Vergleichsbeispiel II |
|---|---|---|
| HDI-Gehalt nach der Lagerung. [Gew.-%] Restliche Bestandteile zu 100 Gew.-%: | 99,67 Oligomere | 98,75 Oligomere |
| NCO-Gehalt der Reaktionsmischung nach der Trimerisierung [Gew.-%] | 39,7 | 39,4 |
| Farbzahl der Reaktionsmischung nach der Trimerisierung [Hazen] | 78 | 145 |

Das Beispiel 2 zeigt, daß die Farbzahl von Isocyanuratgruppen enthaltenden Polyisocyanaten von mit Kohlendioxid stabilisierten HDI-Zusammensetzungen durch die Mitverwendung von 2,6-Di-tert.-butyl-4-methylphenol als zusätzlichen Stabilisator weiter verbessert wurde.

### Beispiel 3

Eine durch thermische Zersetzung von Hexamethylen-dibutylurethan-1,6 hergestellte HDI-Zusammensetzung wurde bei 23°C 5 Minuten mit trockenem Kohlendioxid begast und danach mit 50 mol-ppm 2,6-Di-tert.-butyl-4-methylphenol und 50 mol-ppm Triphenylphosphit versetzt. Die HDI-Zusammensetzung, die einen Kohlendioxidgehalt von ungefähr 1000 ppm besaß, wurde in einem Behälter aus V2A-Stahl bei 5°C gelagert.

### Vergleichsbeispiel III

Ein Teil der vorgenannten HDI-Zusammensetzung wurde lediglich mit 50 mol-ppm 2,6-Di-tert.-butyl-4-methylphenol und 50 mol-ppm Triphenylphosphit stabilisiert und analog Beispiel 3 gelagert.

### Herstellung von Isocyanuratgruppen enthaltenden Polyisocyanatmischungen

Nach einer Lagerzeit von 30 Tagen wurden die HDI-Zusammensetzungen nach Beispiel 3 und Vergleichsbeispiel III in Gegenwart von 500 ppm N,N,N-Trimethyl-N-(2-hydroxypropyl)ammonium-2-ethylhexanoat bei 80°C unter Rühren trimerisiert. Nach einer Stunde wurde die Reaktion durch Zugabe von Dibutylphosphat gestoppt.

An den Reaktionsmischungen wurden die folgenden Meßergebnisse ermittelt:

| | Beispiel 3 | Vergleichsbeispiel II |
|---|---|---|
| HDI-Gehalt nach der Lagerung. [Gew.-%] | > 99,9 | 99,69 |
| Restliche Bestandteile zu 100 Gew.-%: | Oligomere | Oligomere |
| NCO-Gehalt der Reaktionsmischung nach der Trimerisierung [Gew.-%] | 38,3 | 38 |
| Farbzahl der Reaktionsmischung nach der Trimerisierung [Hazen] | 49 | 58 |

Das Beispiel 3 zeigt, daß die Farbzahl von Isocyanuratgruppen enthaltenden Polyisocyanaten von mit Kohlendioxid stabilisierten HDI-Zusammensetzungen durch die Mitverwendung von 2,6-Di-tert.-butyl-4-methylphenol und Triphenylphosphit als zusätzliche Stabilisatoren weiter verbessert wurde.

## Patentansprüche

1. Stabile (cyclo)aliphatische Polyisocyanatzusammensetzungen, enthaltend
i) ein nach einem phosgenfreien Verfahren hergestelltes (cyclo)aliphatisches Polyisocyanat und
ii) Kohlendioxid.

2. Stabile (cyclo)aliphatische Polyisocyanatzusammensetzungen, enthaltend
i) ein durch thermische Spaltung von (cyclo)aliphatischen Polycarbaminsäureestern erhältliches (cyclo)aliphatisches Polyisocyanat und
ii) Kohlendioxid.

3. Stabile (cyclo)aliphatische Polyisocyanatzusammensetzungen nach Anspruch 1 oder 2, enthaltend als aliphatisches Polyisocyanat (i) 1,6-Hexamethylen-diisocyanat.

4. Stabile (cyclo)aliphatische Polyisocyanatzusammensetzungen nach Anspruch 1 oder 2, enthaltend als cycloaliphatisches Polyisocyanat (i) 1-Isocyanato-3-isocyanatomethyl-3,5,5-trimethyl-cyclohexan.

5. Stabile (cyclo)aliphatische Polyisocyanatzusammensetzungen nach Anspruch 1 oder 2, enthaltend Kohlendioxid (ii) in einer Menge bis 0,3 Gew.-%, bezogen auf das Gesamtgewicht.

6. Stabile (cyclo)aliphatische Polyisocyanatzusammensetzungen nach Anspruch 1 oder 2, enthaltend Kohlendioxid (ii) in einer Menge von 0,004 bis 0,3 Gew.-%, bezogen auf das Gesamtgewicht.

7. Verfahren zur Verbesserung der Lagerbeständigkeit von nach einem phosgenfreien Verfahren erhältlichen (cyclo)aliphatischen Polyisocyanatzusammensetzungen, dadurch gekennzeichnet, daß diese einen Kohlendioxidgehalt von 0,004 bis 0,3 Gew.-%, bezogen auf das Gesamtgewicht, besitzen.

8. Verfahren zur Verbesserung der Lagerbeständigkeit von durch thermische Spaltung von (cyclo)aliphatischen Polycarbaminsäureestern erhältlichen (cyclo)aliphatischen Polyisocyanatzusammensetzungen, dadurch gekennzeichnet, daß diese einen Kohlendioxidgehalt von 0,004 bis 0,3 Gew.-%, bezogen auf das Gesamtgewicht besitzen.

9. Verfahren nach Anspruch 8, dadurch gekennzeichnet, daß die Polyisocyanatzusammensetzung als aliphatisches Polyisocyanat 1,6-Hexamethylen-diisocyanat enthält.

10. Verfahren nach Anspruch 8, dadurch gekennzeichnet, daß die Polyisocyanatzusammensetzung als cycloaliphatisches Polyisocyanat 1-Isocyanato-3-isocyanatomethyl-3,5,5-trimethyl-cyclohexan enthält.

## Claims

1. A stable (cyclo)aliphatic polyisocyanate composition containing
i) a (cyclo)aliphatic polyisocyanate prepared by a phosgene-free method and
ii) carbon dioxide.

2. A stable (cyclo)aliphatic polyisocyanate composition containing
i) a (cyclo)aliphatic polyisocyanate obtainable by thermal cleavage of (cyclo)aliphatic polycarbamates and
ii) carbon dioxide.

3. A stable (cyclo)aliphatic polyisocyanate composition as claimed in claim 1 or 2, containing hexamethylene 1,6-diisocyanate as the aliphatic polyisocyanate (i).

4. A stable (cyclo)aliphatic polyisocyanate composition as claimed in claim 1 or 2, containing 1-isocyanato-3-isocyanatomethyl-3,5,5-trimethylcyclohexane as the cycloaliphatic polyisocyanate (i).

5. A stable (cyclo)aliphatic polyisocyanate composition as claimed in claim 1 or 2, containing carbon dioxide (ii) in an amount of up to 0.3% by weight, based on the total weight.

6. A stable (cyclo)aliphatic polyisocyanate composition as claimed in claim 1 or 2, containing carbon dioxide (ii) in an amount of from 0.004 to 0.3% by weight, based on the total weight.

7. A process for improving the shelf-life of (cyclo)aliphatic polyisocyanate compositions obtainable by a phosgene-free method, wherein said compositions have a carbon dioxide content of from 0.004 to 0.3% by weight, based on the total weight.

8. A process for improving the shelf-life of (cyclo)aliphatic polyisocyanate compositions obtainable by thermal cleavage of (cyclo)aliphatic polycarbamates, wherein said compositions have a carbon dioxide content of from 0.004 to 0.3% by weight, based on the total weight.

9. A process as claimed in claim 8, wherein the polyisocyanate composition contains hexamethylene 1,6-diisocyanate as the aliphatic polyisocyanate.

10. A process as claimed in claim 8, wherein the polyisocyanate composition contains 1-isocyanato-3-isocyanatomethyl-3,5,5-trimethylcyclohexane as the cycloaliphatic polyisocyanate.

## Revendications

1. Compositions de polyisocyanates (cyclo)aliphatiques stables, contenant
i) un polyisocyanate (cyclo)aliphatique préparé par un procédé sans phosgène et
ii) de l'anhydride carbonique.

2. Compositions de polyisocyanates (cyclo)aliphatiques stables, contenant
i) un polyisocyanate (cyclo)aliphatique obtenu par dissociation thermique de polycarbamates (cyclo)aliphatiques et
ii) de l'anhydride carbonique.

3. Compositions de polyisocyanates (cyclo)aliphatiques stables selon la revendication 1 ou 2, contenant du diisocyanate de 1,6-hexaméthylène en tant que polyisocyanate aliphatique (i).

4. Compositions de polyisocyanates (cyclo)aliphatiques stables selon la revendication 1 ou 2, contenant du 1-isocyanato-3-isocyanatométhyl-3,5,5-triméthylcyclohexane en tant que polyisocyanate cycloaliphatique (i).

5. Compositions de polyisocyanates (cyclo)aliphatiques stables selon la revendication 1 ou 2, contenant de l'anhydride carbonique (ii) dans une proportion allant jusqu'à 0,3% en poids par rapport au poids total.

6. Compositions de polyisocyanates (cyclo)aliphatiques stables selon la revendication 1 ou 2, contenant de l'anhydride carbonique (ii) dans une proportion de 0,004 à 0,3% en poids par rapport au poids total.

7. Procédé pour améliorer la stabilité au stockage de compositions de polyisocyanates (cyclo)aliphatiques obtenues par un procédé sans phosgène, caractérisé en ce que ces compositions ont une teneur en anhydride carbonique de 0,004 à 0,3% en poids par rapport au poids total.

8. Procédé pour améliorer la stabilité au stockage de compositions de polyisocyanates (cyclo)aliphatiques obtenues par dissociation thermique de polycarbamates (cyclo)aliphatiques, caractérisé en ce que ces compositions ont une teneur en anhydride carbonique de 0,004 à 0,3% en poids par rapport au poids total.

9. Procédé selon la revendication 8, caractérisé en ce que la composition de polyisocyanate contient du diisocyanate de 1,6-hexaméthylène en tant que polyisocyanate aliphatique.

10. Procédé selon la revendication 8, caractérisé en ce que la composition de polyisocyanate contient du 1-isocyanato-3-isocyanatométhyl-3,5,5-triméthylcyclohexane en tant que polyisocyanate cycloaliphatique.
